# DEMANDE DE BREVET EUROPEEN

(11) **EP 4 570 175 A1**
(43) Date de publication de la demande: **18.06.2025**
(21) Numéro de dépôt: 24219650.9
(22) Date de dépôt: 13.12.2024
(51) Int. Cl.: A61B 5/145, A61B 5/1477, A61B 5/1495, A61B 5/00, A61B 10/00

(54) **DISPOSITIF PORTABLE PAR UNE PERSONNE POUR ANALYSER UN FLUIDE BIOLOGIQUE ET PROCÉDÉ DE MISE EN OEUVRE ASSOCIÉ**

(30) Priorité: 14.12.2023 FR 2314184
(71) Demandeur: Commissariat à l'Energie Atomique et aux Energies Alternatives, 75015 Paris (FR)
(72) Inventeur: THOMAS, Yohann, 38054 Grenoble (FR); MAILLEY, Pascal, 38054 Grenoble (FR)
(74) Mandataire: Gevers & Orès

(57) **Abrégé**

L'invention concerne un dispositif (D) portable par une personne pour analyser un fluide biologique, comprenant :
- une zone de collecte (ZC) d'un fluide biologique,
- une zone de mesure (ZM) comportant des capteurs électrochimiques (CEC),
- une zone de transport (ZT) pour le fluide biologique, reliant la zone de collecte (ZC) à la zone de mesure (ZM),
- au moins une cartouche (CART1) remplie d'un fluide d'étalonnage, ladite au moins une cartouche étant agencée pour que le fluide d'étalonnage puisse parvenir jusqu'à la zone de mesure (ZM), et
- une zone d'évacuation (ZEV) du fluide susceptible d'être présent dans la zone de mesure (ZM), ladite zone d'évacuation (ZEV) comportant une zone réservoir (ZRES) munie d'un matériau absorbant,
ladite au moins une cartouche (CART1) est montée au contact de la zone de transport (ZT) par l'intermédiaire d'un opercule (OPC1) ouvrable sous l'effet d'une pression.

## Description

### Domaine technique de l'invention

L'invention se réfère aux dispositifs pour l'analyse de fluides biologiques pouvant être portés sur une personne. Elle concerne en particulier un dispositif, susceptible d'être porté par une personne, muni de capteurs électrochimiques pour analyser les fluides biologiques. Plus spécifiquement, l'invention se réfère à la mise en oeuvre de ces dispositifs et plus particulièrement à leur étalonnage.

### Arrière-plan technique

Les capteurs électrochimiques sont des dispositifs qui convertissent les signaux chimiques en signaux électriques mesurables. Leur principe de base repose sur les réactions électrochimiques qui se produisent à l'interface entre un matériau sensible et une solution chimique. Ces réactions génèrent un courant électrique, une différence de potentiel ou une variation d'impédance électrochimique, qui peuvent être mesurés et utilisés pour quantifier la concentration d'une substance cible. Il existe différents types de capteurs électrochimiques, tels que les électrodes à oxygène, les électrodes pH, les électrodes sensibles aux ions sélectifs ou encore les électrodes sensibles à des espèces chimiques ou biologiques spécifiques, chaque type d'électrode utilisera un élément sensible spécifique.

Les avantages des capteurs électrochimiques sont leur sensibilité élevée, leur sélectivité et leur temps de réponse court. Ils sont également relativement compacts, facilement intégrables, peu coûteux et faciles à utiliser. Ils sont par conséquent largement utilisés dans de nombreux domaines, tels que la surveillance de la qualité de l'eau, l'analyse des gaz, le contrôle des processus industriels et les dispositifs médicaux.

Les capteurs électrochimiques peuvent donc être naturellement appliqués à l'analyse de tout type de fluide biologique, en particulier à celle de la sueur. En effet, la sueur contient diverses substances, parmi lesquelles des électrolytes, des métabolites ou des biomarqueurs, qui peuvent fournir des informations sur l'état de santé, l'hydratation, l'activité métabolique et d'autres paramètres physiologiques de la personne portant le capteur. A titre d'exemple, nous pouvons mentionner le glucose, les ions sodium et potassium, le lactate ou d'autres substances chimiques ou biochimiques pertinentes.

Les capteurs électrochimiques pour l'analyse de la sueur peuvent être portables et non invasifs, ce qui les rend pratiques pour surveiller en temps réel les paramètres de santé lors d'activités physiques ou dans le cadre de soins médicaux. Ils peuvent également être intégrés à des dispositifs portables, tels que des bracelets ou des patchs, pour permettre un suivi en temps réel, continu et à long terme.

Il convient de noter que les capteurs électrochimiques appliqués au monitoring de la sueur sont en développement continu. La recherche dans ce domaine vise à améliorer la précision, la sensibilité et la fiabilité de ces capteurs, ainsi qu'à élargir la gamme d'analytes pouvant être mesurés.

Un point d'attention à porter aux capteurs électrochimiques et plus particulièrement à ceux visant à analyser la sueur d'une personne est l'étalonnage. L'étalonnage est en effet une étape nécessaire pour assurer la précision et la fiabilité des mesures. En général, plusieurs actions sont nécessaires et communément appliquées en laboratoire : préparation des solutions d'étalonnage, nettoyage du capteur, réglage du zéro, étalonnage, établissement de la courbe d'étalonnage, vérification et ajustement final (avec par exemple une vérification de la linéarité et de la précision).

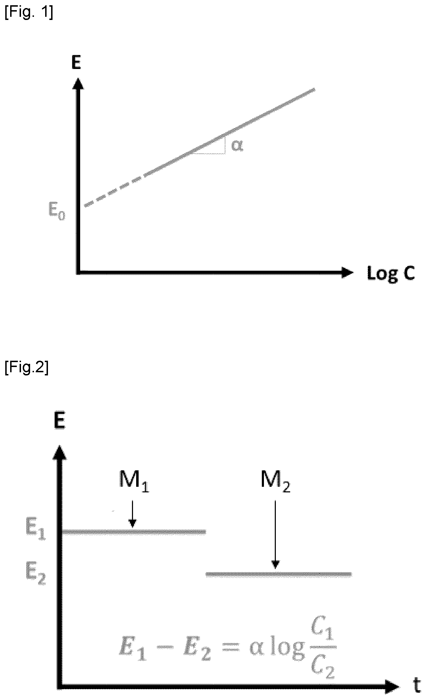

Sur la figure 1 on présente le cas d'une courbe de calibration d'un capteur électrochimique de type potentiométrique qui se définit par la relation [Maths1] *E* = *E*₀ + *α.* log(*C*), où *E* est la différence de potentiel mesuré, C est la concentration en analyte, *E₀* est un paramètre de calibration (parfois appelé « *offset » en* anglais) et *α* est la sensibilité du capteur (exprimés selon cette équation en unité de potentiel par unité de décade de concentration).

Une possibilité est d'effectuer un étalonnage proche de la mesure avec une mesure unique dans un fluide d'étalonnage (référence) mimant le milieu à analyser à une concentration suffisamment voisine de la concentration cible. Cela permet de considérer que la sensibilité *α* est identique entre le fluide d'étalonnage et le milieu à analyser mais de corriger la valeur *E₀*. Afin de mieux expliciter cette stratégie, nous représentons sur la figure 2, un exemple de mesure (mesures de E₁, E₂) qui fait passer en premier un étalon M₁ (dont la concentration C₁ de l'analyte d'intérêt est parfaitement connue) et l'échantillon M₂ (« *sample »* en anglais) dont la concentration C₂ est à déterminer. Cette approche permet de s'affranchir de la valeur de E₀. Il convient de connaitre la valeur de la sensibilité *α* laquelle est généralement établie par batch de capteurs fabriqués. Cette approche a ses limites car au fil du temps, la valeur de la sensibilité *α* peut évoluer.

Une autre possibilité est d'effectuer un étalonnage proche de la mesure avec des mesures multiples (au moins deux) avec différents fluides d'étalonnage. Ceci permet en plus de corriger la valeur de la sensibilité *α.* Cette approche peut être particulièrement adaptée dans un milieu complexe comme les fluides biologiques (sueur par exemple) car il peut y avoir présence de molécules interférentes qui vont entrainer une surestimation de la concentration mesurée. Par ailleurs certains paramètres physicochimiques tels que la conductivité, le pH ou la température peuvent impacter la mesure.

Quelle que soit le type d'étalonnage considéré, celui-ci est de type laboratoire c'est-à-dire qu'il est fait avant la mesure effective.

Dans le domaine particulier de l'analyse de la sueur, on peut par exemple citer l'étude de Parrilla et al., « Wearable Potentiometric Ion Patch for On-Body Electrolyte Monitoring in Sweat: Toward a Validation Strategy to Ensure Physiological Relevance », Analytical Chemistry 2019, vol. 91, pp. 8644-8651. (https://pubs.acs.org/doi/pdf/10.1021/acs.analchem.9b02126). Cette étude propose un dispositif de capteurs électrochimiques pour le monitoring potentiométrique du pH, Cl⁻, K⁺ et Na⁺, dispositif qui est apte à être porté par une personne. Ce dispositif comprend une cellule flexible de collecte couplée à une cellule d'analyse. Le dispositif est intéressant car il est stable (faible dérive), avec un temps de réponse rapide, des sélectivités adéquates pour la mesure de la sueur et une très bonne réversibilité. Par ailleurs, la conception du dispositif permet d'éviter une contamination de la sueur, les problèmes liés à l'évaporation, et le flux passif de sueur représente correctement la perspiration. Néanmoins, les calibrations des capteurs électrochimiques sont faites avant l'utilisation, au laboratoire. Les auteurs ont réalisé des points de contrôle pendant la mesure, mais en réalisant une mesure externe des analytes d'intérêts sur de la sueur extraite après prélèvement.

Un objectif de l'invention est de proposer un dispositif muni de capteurs électrochimiques, aptes à être portés par une personne, ne présentant pas l'un au moins des inconvénients précités.

Plus précisément, un objectif de l'invention est de proposer un dispositif muni de capteurs électrochimiques, aptes à être portés par une personne, les capteurs électrochimiques étant par exemple capables d'effectuer de la mesure sur un fluide biologique comme la sueur, et permettant dans le même temps un étalonnage des capteurs électrochimiques sans prélèvement destiné à effectuer une analyse externe au dispositif.

### Résumé de l'invention

Pour résoudre l'objectif précité, l'invention propose un dispositif portable par une personne pour analyser un fluide biologique, comprenant :
- une zone de collecte d'un fluide biologique comme la sueur,
- une zone de mesure comportant une pluralité de capteurs électrochimiques,
- une zone de transport pour le fluide biologique, reliant la zone de collecte à la zone de mesure, le dispositif étant caractérisé en ce qu'il comprend :

- au moins une cartouche remplie d'un fluide d'étalonnage, ladite au moins une cartouche étant agencée pour que le fluide d'étalonnage puisse parvenir jusqu'à la zone de mesure, et
- une zone d'évacuation du fluide susceptible d'être présent dans la zone de mesure, ladite zone d'évacuation comportant une zone réservoir munie d'un matériau absorbant.

L'invention propose donc une solution innovante pour améliorer la précision et la fiabilité de la mesure obtenue par des capteurs électrochimiques intégrés dans un dispositif susceptible d'être porté par une personne pour l'analyse de fluides biologiques comme la sueur.

Comme on peut le comprendre, l'étalonnage s'effectue in situ sans qu'un quelconque prélèvement ne soit effectué dans le dispositif pour une analyse externe, de type laboratoire.

Le dispositif selon l'invention pourra comprendre l'une au moins des caractéristiques suivantes, prises seules ou en combinaison :
- plusieurs cartouches chacune munie d'un fluide d'étalonnage, chaque cartouche étant agencée pour que le fluide d'étalonnage puisse parvenir jusqu'à la zone de mesure,
- deux cartouches au moins de ladite pluralité de cartouches comportent un fluide d'étalonnage différent ;
- ladite au moins une cartouche ou, le cas échéant, chaque cartouche, est montée au contact de la zone de transport par l'intermédiaire d'un opercule ouvrable sous l'effet d'une pression.

L'invention concerne également un procédé de mise en oeuvre d'un dispositif selon l'une des revendications précédentes, caractérisé en ce qu'il comprend les étapes suivantes :
a) installer le dispositif sur une personne en mettant la zone de collecte contre sa peau,
b) attendre que la zone de mesure soit immergée d'un échantillon de fluide biologique, par exemple de la sueur,
c) effectuer une mesure sur l'échantillon de fluide biologique, au niveau de la zone de mesure,
d) évacuer le fluide biologique de la zone de mesure vers la zone d'évacuation de fluide,
e) exercer une pression contre la cartouche pour libérer un fluide d'étalonnage dans la zone de transport afin qu'elle rejoigne la zone de mesure,
f) attendre que la zone de mesure soit immergée dans le fluide d'étalonnage,
g) effectuer un étalonnage au niveau de la zone de mesure, et
h) évacuer le fluide d'étalonnage de la zone de mesure, dans lequel soit les étapes a) à h) sont mises en oeuvre à la suite, soit les étapes e), f), g) et h) sont d'abord mises en oeuvre à la suite et ensuite suivies par les étapes a), b), c) puis d).

Le procédé selon l'invention pourra comprendre l'une au moins des étapes suivantes, prise seule ou en combinaison :
- après avoir mis en oeuvre l'ensemble des étapes a) à h), le procédé comprend l'étape suivante : i) répéter N fois les étapes b), c) et d) pour effectuer une mesure sur N autre(s) échantillon(s) biologique(s) où N est un entier naturel supérieur ou égal à l'unité ;
- le dispositif comprenant plusieurs cartouches contenant des fluides d'étalonnage identiques, le procédé comprend, après avoir mis en oeuvre l'étape i), les étapes suivantes :
   e₁') exercer une pression sur une autre cartouche pour libérer un autre fluide d'étalonnage dans la zone de transport afin qu'elle rejoigne la zone de mesure, cet autre fluide d'étalonnage étant identique au fluide d'étalonnage employé au cours de l'étape e),
   f₁') attendre que la zone de mesure soit immergée dans le fluide d'étalonnage, et
   g₁') effectuer un autre étalonnage au niveau de la zone de mesure.
- le dispositif comprenant plusieurs cartouches contenant des fluides d'étalonnage différents, le procédé comprend les étapes additionnelles suivantes, mises en oeuvre directement à la suite de l'étape h) :
   e₁") exercer une pression contre une autre cartouche pour libérer un autre fluide d'étalonnage dans la zone de transport afin qu'elle rejoigne la zone de mesure, cet autre fluide d'étalonnage étant différent du fluide d'étalonnage employé au cours de l'étape e),
   f₁") attendre que la zone de mesure soit immergée dans cet autre fluide d'étalonnage,
   g₁") effectuer une mesure sur cet autre fluide d'étalonnage, au niveau de la zone de mesure ;
- après avoir mis en oeuvre l'étape g₁"), le procédé comprend l'étape suivante : i") répéter N fois les étapes b), c) et d) pour effectuer une mesure sur N autre(s) échantillon(s) biologique(s) où N est un entier naturel supérieur ou égal à l'unité ;
- après la mise en oeuvre de l'étape i"), le procédé comprend les étapes suivantes :
   e₂') exercer une pression sur une autre cartouche pour libérer un autre fluide d'étalonnage dans la zone de transport afin qu'elle rejoigne la zone de mesure, cet autre fluide d'étalonnage étant identique au fluide d'étalonnage employé au cours de l'étape e),
   f'₂') attendre que la zone de mesure soit immergée dans le fluide d'étalonnage, et
   g₂') effectuer un autre étalonnage au niveau de la zone de mesure ;
- après avoir évacué l'autre fluide d'étalonnage de la zone de mesure à l'issue de la mise en oeuvre de l'étape g₂'), le procédé comprend les étapes suivantes :
   e₂") exercer une pression contre une autre cartouche pour libérer encore un autre fluide d'étalonnage dans la zone de transport afin qu'elle rejoigne la zone de mesure, cet autre fluide d'étalonnage étant différent du fluide d'étalonnage employé au cours de l'étape e),
   f₂") attendre que la zone de mesure soit immergée par cet autre fluide d'étalonnage,
   g₂") effectuer une mesure sur cet autre fluide d'étalonnage, au niveau de la zone de mesure ;
- le fluide d'étalonnage employé à l'étape e₂") est identique au fluide d'étalonnage employé à l'étape e₁").

### Brève description des figures

D'autres objets et caractéristiques de l'invention apparaîtront plus clairement dans la description qui suit, faite en référence aux figures annexées, dans lesquelles :
La [Fig. 3] représente un premier mode de réalisation d'un dispositif portable par une personne pour analyser un fluide biologique conforme à l'invention ;
La [Fig. 4] représente une variante du premier mode de réalisation représenté sur la [Fig. 3] ;
La [Fig. 5] représente un procédé de mise en oeuvre du dispositif de la [Fig. 3] ou de la [Fig. 4], dans lequel on effectue une mesure sur le fluide biologique puis un étalonnage ;
La [Fig. 6] représente un autre procédé de mise en oeuvre du dispositif de la [Fig. 3] ou de la [Fig. 4], dans lequel on effectue un étalonnage puis une mesure sur le fluide biologique ;
La [Fig. 7] représente un deuxième mode de réalisation d'un dispositif portable par une personne pour analyser un fluide biologique conforme à l'invention.

### Description détaillée de l'invention

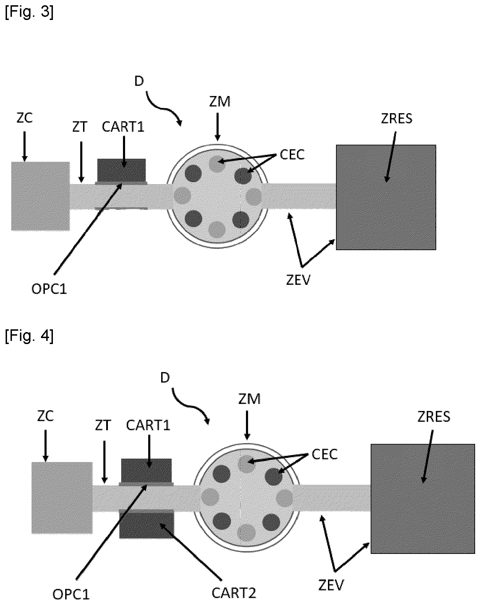

Sur la figure 3, on a représenté un premier mode de réalisation d'un dispositif conforme à l'invention.

Le dispositif D est un dispositif portable par une personne pour analyser un fluide biologique. Le dispositif D comprend une zone de collecte ZC d'un fluide biologique. La zone de collecte SC est destinée à venir au contact de la peau de la personne et comporte avantageusement un adhésif pour coller à la peau. Le fluide biologique peut notamment être la sueur de la personne par laquelle le dispositif D est porté, sueur naturellement dégagée lors d'une activité physique. Le dispositif D comprend aussi une zone de mesure ZM comportant une pluralité de capteurs électrochimiques CEC. Le dispositif D comprend également une zone de transport ZT pour le fluide, reliant la zone de collecte ZC à la zone de mesure ZM.

Le dispositif D comprend par ailleurs au moins une cartouche CART1 remplie d'un fluide d'étalonnage. Ladite au moins une cartouche est agencée pour que le fluide d'étalonnage puisse parvenir jusqu'à la zone de mesure ZM. A cet effet, et dans ce mode de réalisation, la cartouche CART1 est montée au contact de la zone de transport ZT par l'intermédiaire d'un opercule OPC1 ouvrable sous l'effet d'une pression par exemple du fait de l'action mécanique d'une personne sur la cartouche ou par une action automatisée (mécanique, thermique, électrique ou autre) sur la cartouche. La zone de transport ZT est donc susceptible de transporter le fluide biologique prélevé comme le fluide d'étalonnage vers la zone de mesure ZM.

Le dispositif D comprend enfin une zone d'évacuation ZEV du fluide susceptible d'être présent dans la zone de mesure ZM. Cette zone d'évacuation ZEV de fluide permet notamment de renouveler l'échantillon de fluide biologique présent dans la zone de mesure pour effectuer d'autres mesures, ou d'évacuer le fluide biologique pour y insérer un fluide d'étalonnage ou inversement. Par ailleurs, la zone d'évacuation ZEV du fluide comporte une zone réservoir ZRES faite d'un matériau absorbant, par exemple un superabsorbant. Un superabsorbant est typiquement constitué d'un matériau poreux aux capacités d'absorption d'eau nettement plus élevée que celles de la zone de transport ZT (veine fluidique), sa taille étant fixée par le flux de sudation et la durée de mise en oeuvre. On permet ainsi le transport monodirectionnel puis l'évacuation du fluide présent dans la zone de mesure ZM sans accumulation d'analytes au cours du temps.

Dans la variante de réalisation de la figure 4, le dispositif D comprend plusieurs cartouches CART1, CART2 chacune munie d'un fluide d'étalonnage. Dans cette variante, chaque cartouche CART1, CART2 est montée au contact de la zone de transport ZT par l'intermédiaire d'un opercule OPC1 ouvrable sous l'action de pressage exercée sur la cartouche par une personne.

On peut alors prévoir que les cartouches CART1, CART2 contiennent un fluide d'étalonnage identique. Cela permet alors d'effectuer plusieurs étalonnages avec le même dispositif D, pour ajuster ou s'affranchir du paramètre de calibration Eo des capteurs électrochimiques (cf. figure 2) à plusieurs reprises au cours des mesures. Par exemple, il est alors possible d'effectuer un étalonnage avant toute prise de mesures sur un échantillon de fluide biologique et de faire un autre étalonnage après une ou quelques prises de mesures sur un ou d'autre(s) échantillon(s) de fluide biologique. En particulier, il est alors possible d'effectuer un étalonnage avant toute prise de mesures sur un échantillon de fluide biologique et de faire un autre étalonnage après avoir terminé ladite prise de mesures, si bien qu'on est alors prêt à engager une autre série de mesures ultérieurement.

Au contraire, les cartouches CART1, CART2 peuvent contenir un fluide d'étalonnage différent. Cela permet alors d'effectuer un étalonnage avec le même dispositif D, pour à la fois ajuster ou s'affranchir du paramètre de calibration Eo et ajuster la sensibilité α des capteurs électrochimiques (figure 2). En particulier, il est alors possible d'effectuer un tel étalonnage avant toute prise de mesures sur un échantillon de fluide biologique. Bien entendu, et en variante, cet étalonnage peut s'effectuer entre deux séries de mesures ou en toute fin d'une prise de mesures.

On notera qu'il est possible de prévoir bien plus que deux cartouches en fonction des besoins et par ailleurs que ces cartouches peuvent contenir des fluides d'étalonnage identiques ou différents. Dans ce dernier cas, les fluides d'étalonnage peuvent en particulier être tous différents. Ainsi, si on envisage par exemple trois cartouches, on peut prévoir trois fluides d'étalonnage différentes pour une meilleure estimation de la sensibilité des capteurs avec des fluides corporels, qui peuvent être particulièrement complexes à analyser.

Comme on peut le constater sur les figures 3 (premier mode de réalisation) et 4 (variante au premier mode de réalisation), un fluide qu'il soit biologique ou pour l'étalonnage se propage de manière linéaire le long du dispositif D. D'un point de vue pratique, une personne peut monter ce type de dispositif sur son bras, avec un bracelet autour du poignet par exemple, la propagation se faisant alors typiquement le long de l'axe du bras.

### Exemple de réalisation.

La zone de collecte ZC peut par exemple se présenter sous la forme d'un tampon collecteur ou d'un adhésif silicone.

La zone de transfert ZT se présente par exemple sous la forme d'un canal fluidique réalisé dans un matériau polymère (il peut être réalisé par usinage, moulage ou thermoformage, etc) ou de type papier.

La partie canal fluidique de la zone d'évacuation de fluide ZEV peut être réalisé de manière analogue à la zone de transfert ZT. Quant à la zone réservoir ZREV de la zone d'évacuation de fluide, on peut choisir un matériau superabsorbant, en un matériau synthétique comme le polyacrylate de sodium, le polyacrylonitrile, un non-tissé, ou du papier, ou en un matériau naturel comme le coton ou la sphaigne.

Enfin, la zone de mesure ZM est une chambre fluidique qui peut être obtenue par formation physique d'un polymère (usinage, thermoformage, moulage notamment) ou de type papier. Ses dimensions et sa forme sont adaptés aux nombres de mesures requises et à leur redondance.

### Fin de l'exemple.

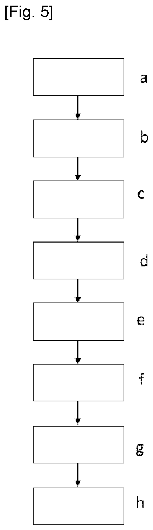

Sur la figure 5, on a représenté différentes étapes d'un procédé de mise en oeuvre d'un dispositif D conforme à l'invention, tel que celui qui est représenté sur la figure 3 ou sur la figure 4.

Dans le procédé représenté schématiquement sur la figure 5, on effectue une mesure sur le fluide biologique puis on effectue un étalonnage.

Plus précisément, le procédé comprend les étapes suivantes :
a) installer le dispositif D sur une personne en mettant la zone de collecte ZC contre sa peau,
b) attendre que la zone de mesure ZM soit immergée d'un échantillon de fluide biologique, par exemple de la sueur,
c) effectuer une mesure sur l'échantillon de fluide biologique, au niveau de la zone de mesure ZM,
d) évacuer le fluide biologique de la zone de mesure ZM vers la zone d'évacuation ZEV de fluide,
e) exercer une pression sur la cartouche CART1 pour libérer le fluide d'étalonnage dans la zone de transport ZT afin qu'elle rejoigne la zone de mesure ZM,
f) attendre que la zone de mesure ZM soit immergée par le fluide d'étalonnage,
g) effectuer un étalonnage au niveau de la zone de mesure ZM, et
h) évacuer le fluide d'étalonnage de la zone de mesure ZM.

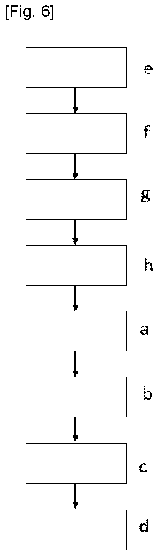

Sur la figure 6, on a représenté différentes étapes d'un autre procédé de mise en oeuvre d'un dispositif D conforme à l'invention, tel que celui représenté sur la figure 3 ou sur la figure 4.

Dans le procédé représenté schématiquement sur la figure 6, on effectue un étalonnage puis on effectue une mesure sur le fluide biologique.

Plus précisément, le procédé comprend les étapes suivantes :
e) exercer une pression sur la cartouche CART1 pour libérer le fluide d'étalonnage dans la zone de transport ZT afin qu'elle rejoigne la zone de mesure ZM,
f) attendre que la zone de mesure ZM soit immergée par le fluide d'étalonnage,
g) effectuer un étalonnage au niveau de la zone de mesure ZM, et
h) évacuer le fluide d'étalonnage de la zone de mesure ZM, puis

a) installer le dispositif D sur une personne en mettant la zone de collecte ZC contre sa peau,
b) attendre que la zone de mesure ZM soit immergée d'un échantillon de fluide biologique, par exemple de la sueur,
c) effectuer une mesure sur l'échantillon de fluide biologique, au niveau de la zone de mesure ZM,
d) évacuer le fluide biologique de la zone de mesure ZM vers la zone d'évacuation ZEV de fluide.

Quelle que soit la variante de mise en oeuvre envisagée, après avoir mis en oeuvre l'ensemble des étapes a) à h) ou e) à h) puis a) à d), on peut mettre en oeuvre l'étape suivante : i) répéter N fois les étapes b), c) et d) pour effectuer une mesure sur N autre(s) échantillon(s) biologique(s) où N est un entier naturel supérieur ou égal à l'unité.

Par ce biais, on effectue plusieurs mesures avec divers échantillons de fluide biologique, avant étalonnage (procédé de la figure 5) ou après étalonnage (procédé de la figure 6).

Par ailleurs, lorsque le dispositif D comprend plusieurs cartouches CART1, CART2 (figure 4) contenant des fluides d'étalonnage identiques, le procédé comprend les étapes suivantes, après avoir mis en oeuvre l'étape i) : e₁') exercer une pression sur une autre cartouche CART2 pour libérer un autre fluide d'étalonnage dans la zone de transport ZT afin qu'elle rejoigne la zone de mesure ZM, cet autre fluide d'étalonnage étant identique au fluide d'étalonnage employé au cours de l'étape e),
f₁') attendre que la zone de mesure ZM soit immergée dans le fluide d'étalonnage, et
g₁') effectuer un autre étalonnage au niveau de la zone de mesure ZM.

Cela permet de mettre en oeuvre un autre étalonnage du même type que celui qui a été effectué précédemment lors de l'étape g).

Lorsque le dispositif comprend plusieurs cartouches CART1, CART2 (figure 4) contenant des fluides d'étalonnage différents, le procédé peut comprendre les étapes additionnelles suivantes, mises en oeuvre à la suite de l'étape h) (à savoir juste après l'étape h) qu'un étalonnage selon l'étape g) ait été fait avant ou après une mesure sur un fluide biologique) : e₁") exercer une pression contre une autre cartouche CART2 pour libérer un autre fluide d'étalonnage dans la zone de transport ZT afin qu'elle rejoigne la zone de mesure ZM, cet autre fluide d'étalonnage étant différent du fluide d'étalonnage employé au cours de l'étape e), f₁") attendre que la zone de mesure ZM soit immergée dans cet autre fluide d'étalonnage, g₁") effectuer une mesure sur cet autre fluide d'étalonnage, au niveau de la zone de mesure ZM.

Par ce biais, on effectue un étalonnage avec au moins deux fluides d'étalonnage différents. On peut ainsi apporter une correction ou s'affranchir du paramètre de calibration Eo et ajuster la sensibilité α des capteurs électrochimiques.

Après cet étalonnage, on peut bien entendu effectuer d'autres mesures sur d'autres échantillons de fluide biologique. Ainsi, le procédé peut comprendre l'étape suivante, après avoir mis en oeuvre l'étape g₁") : i") répéter N fois les étapes b), c) et d) pour effectuer une mesure sur N autre(s) échantillon(s) biologique(s) où N est un entier naturel supérieur ou égal à l'unité.

Ultérieurement, après la mise en oeuvre de l'étape i"), il est envisageable de prévoir un autre étalonnage en mettant en oeuvre les étapes suivantes : e₂') exercer une pression sur une autre cartouche CART2 pour libérer un autre fluide d'étalonnage dans la zone de transport ZT afin qu'elle rejoigne la zone de mesure ZM, cet autre fluide d'étalonnage étant identique au fluide d'étalonnage employé au cours de l'étape e),
f'₂') attendre que la zone de mesure ZM soit immergée dans le fluide d'étalonnage, et
g₂') effectuer un autre étalonnage au niveau de la zone de mesure ZM.

Ici, il s'agit alors seulement d'effectuer un étalonnage pour ajuster ou s'affranchir du paramètre de calibration Eo et donc corriger une éventuelle dérive des capteurs électrochimiques à cet égard, en supposant donc que la sensibilité α des capteurs électrochimiques n'a pas évolué.

Au contraire ou en complément, il est cependant envisageable d'en profiter pour corriger aussi la sensibilité α des capteurs électrochimiques. Dans ce but, après avoir évacué l'autre fluide d'étalonnage de la zone de mesure à l'issue de la mise en oeuvre de l'étape g₂'), les étapes suivantes peuvent être mises en oeuvre: e₂") exercer une pression contre une autre cartouche CART4 pour libérer encore un autre fluide d'étalonnage dans la zone de transport ZT afin qu'elle rejoigne la zone de mesure ZM, cet autre fluide d'étalonnage étant différent du fluide d'étalonnage employé au cours de l'étape e),
f₂") attendre que la zone de mesure ZM soit immergée par cet autre fluide d'étalonnage,
g₂") effectuer une mesure sur cet autre fluide d'étalonnage, au niveau de la zone de mesure ZM.

Le fluide d'étalonnage employé à l'étape e₂") peut être identique au fluide d'étalonnage employé à l'étape e₁"). On peut alors effectuer un autre étalonnage à la fois sur la constante de calibration et sur la sensibilité α des capteurs électrochimiques.

Au contraire, à l'étape e₂"), l'autre cartouche CART4 peut contenir un fluide différent de celui employé à l'étape e₁").

D'une manière générale, un double étalonnage permet de déterminer *E₀* et α*.* Si le double étalonnage est effectué de façon distante dans le temps, il permet de qualifier la dérive du capteur. Le fait d'ajouter des cartouches permet de suivre la dérive sur le plus long terme.

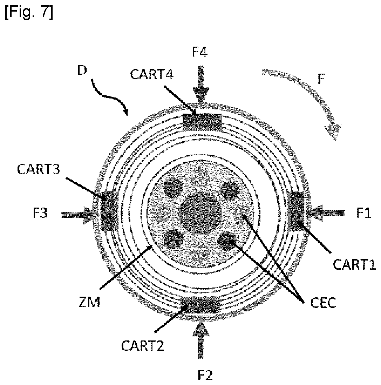

### Un deuxième mode de réalisation est représenté sur la figure 7.

On retrouve ici les mêmes éléments que dans le premier mode de réalisation de l'invention, à savoir une zone de collecte ZC pour un fluide biologique (la figure 7 étant une vue de dessus, la zone de collecte en dessous n'est pas visible), une zone de mesure ZM munie de capteurs électrochimiques CEC, une zone de transfert ZT de fluide vers la zone de mesure ZM, une zone d'évacuation ZEV des fluides (non représentée sur la figure 7 pour avoir une visibilité sur la zone de mesure ZM, mais située au-dessus de la zone de mesure). Par ailleurs, une ou plusieurs cartouches CART1, CART2, CART3, CART4 (en l'occurrence et à titre de simple exemple illustratif, quatre cartouches sur la figure), la ou chaque cartouche contenant un fluide d'étalonnage, sont montées sur la zone de transfert ZT, par exemple par l'intermédiaire d'un opercule OPC1 (représenté uniquement pour la cartouche CART1), entre la zone de collecte ZC et la zone de mesure ZM.

L'originalité de cette conception réside dans la conformation de la zone de transfert ZT qui présente une forme spiralée, la zone de collecte ZC et la zone de mesure ZM étant alors situées en partie centrale l'une au-dessus de l'autre. Une zone réservoir (appartenant à la zone d'évacuation des fluides donc non visible sur la figure 7) de type superabsorbant peut être installée au-dessus du plan de la figure, contre la zone de mesure ZM.

Sur la figure 7, on notera que la flèche F représente le sens de parcours du fluide au sein de la zone de transfert ZT. Les flèches F1, F2, F3 et F4 représentes les actions possibles d'une personne pour presser la cartouche concernée et libérer le fluide d'étalonnage qu'elle contient en perçant l'opercule OPC1.

Cette conformation présente la forme d'une montre et peut donc être aisément portée par une personne lors d'un effort physique au moyen d'un réceptacle de forme circulaire monté sur un bracelet.

Il est à noter que quel que soit le mode de réalisation considéré, le dispositif pourra intégrer un capteur de température pour mesurer la température du fluide prélevé afin d'améliorer la qualité de la mesure.

## Revendications

1. Dispositif (D) destiné à être porté sur une personne pour analyser un fluide biologique, comprenant :
- une zone de collecte (ZC) d'un fluide biologique comme la sueur,
- une zone de mesure (ZM) comportant une pluralité de capteurs électrochimiques (CEC),
- une zone de transport (ZT) pour le fluide biologique, reliant la zone de collecte (ZC) à la zone de mesure (ZM),
- au moins une cartouche (CART1) remplie d'un fluide d'étalonnage, ladite au moins une cartouche étant agencée pour que le fluide d'étalonnage puisse parvenir jusqu'à la zone de mesure (ZM), et
- une zone d'évacuation (ZEV) du fluide susceptible d'être présent dans la zone de mesure (ZM), ladite zone d'évacuation (ZEV) comportant une zone réservoir (ZRES) munie d'un matériau absorbant,
**caractérisé en ce que** ladite au moins une cartouche (CART1) est montée au contact de la zone de transport (ZT) par l'intermédiaire d'un opercule (OPC1) ouvrable sous l'effet d'une pression.

2. Dispositif (D) portable par une personne pour analyser un fluide biologique selon la revendication précédente, **caractérisé en ce qu'**il comprend plusieurs cartouches (CART1, CART2, CART3, CART4) chacune munie d'un fluide d'étalonnage, chaque cartouche étant agencée pour que le fluide d'étalonnage puisse parvenir jusqu'à la zone de mesure (ZM).

3. Dispositif (D) portable par une personne pour analyser un fluide biologique selon la revendication précédente, **caractérisé en ce que** deux cartouches au moins de ladite pluralité de cartouches (CART1, CART2, CART3, CART4) comportent un fluide d'étalonnage différent.

4. Dispositif (D) portable par une personne pour analyser un fluide biologique selon l'une quelconque des revendications 2 ou 3, **caractérisé en ce que** chaque cartouche (CART1, CART2, CART3, CART4), est montée au contact de la zone de transport (ZT) par l'intermédiaire d'un opercule (OPC1) ouvrable sous l'effet d'une pression.

5. Procédé de mise en oeuvre d'un dispositif (D) selon l'une des revendications précédentes, **caractérisé en ce qu'**il comprend les étapes suivantes :
a) installer le dispositif (D) sur une personne en mettant la zone de collecte (ZC) contre sa peau,
b) attendre que la zone de mesure (ZM) soit immergée d'un échantillon de fluide biologique, par exemple de la sueur,
c) effectuer une mesure sur l'échantillon de fluide biologique, au niveau de la zone de mesure (ZM),
d) évacuer le fluide biologique de la zone de mesure (ZM) vers la zone d'évacuation (ZEV) de fluide,
e) exercer une pression contre la cartouche (CART1) pour libérer un fluide d'étalonnage dans la zone de transport (ZT) afin qu'il rejoigne la zone de mesure (ZM),
f) attendre que la zone de mesure (ZM) soit immergée dans le fluide d'étalonnage,
g) effectuer un étalonnage au niveau de la zone de mesure (ZM), et
h) évacuer le fluide d'étalonnage de la zone de mesure (ZM),
dans lequel soit les étapes a) à h) sont mises en oeuvre dans cet ordre, soit les étapes e), f), g) et h) sont d'abord mises en oeuvre dans cet ordre et ensuite suivies par les étapes a), b), c) puis d) dans cet ordre.

6. Procédé selon la revendication précédente, **caractérisé en ce qu'**il comprend, après avoir mis en oeuvre l'ensemble des étapes a) à h), l'étape suivante :
i) répéter N fois les étapes b), c) et d) pour effectuer une mesure sur N autre(s) échantillon(s) biologique(s) où N est un entier naturel supérieur ou égal à l'unité.

7. Procédé selon la revendication précédente, **caractérisé en ce que** le dispositif (D) comprenant plusieurs cartouches (CART1, CART2) contenant des fluides d'étalonnage identiques, le procédé comprend, après avoir mis en oeuvre l'étape i), les étapes suivantes :
e₁') exercer une pression sur une autre cartouche (CART2) pour libérer un autre fluide d'étalonnage dans la zone de transport (ZT) afin qu'elle rejoigne la zone de mesure (ZM), cet autre fluide d'étalonnage étant identique au fluide d'étalonnage employé au cours de l'étape e),
f₁') attendre que la zone de mesure (ZM) soit immergée dans le fluide d'étalonnage, et
g₁') effectuer un autre étalonnage au niveau de la zone de mesure (ZM).

8. Procédé selon la revendication 5, **caractérisé en ce que**, le dispositif (D) comprenant plusieurs cartouches (CART1, CART3) contenant des fluides d'étalonnage différents, le procédé comprend les étapes additionnelles suivantes, mises en oeuvre directement à la suite de l'étape h) :
e₁") exercer une pression contre une autre cartouche (CART3) pour libérer un autre fluide d'étalonnage dans la zone de transport (ZT) afin qu'elle rejoigne la zone de mesure (ZM), cet autre fluide d'étalonnage étant différent du fluide d'étalonnage employé au cours de l'étape e),
f₁") attendre que la zone de mesure (ZM) soit immergée dans cet autre fluide d'étalonnage,
g₁") effectuer une mesure sur cet autre fluide d'étalonnage, au niveau de la zone de mesure (ZM).

9. Procédé selon la revendication précédente, **caractérisé en ce qu'**il comprend, après avoir mis en oeuvre l'étape g₁"), l'étape suivante :
i") répéter N fois les étapes b), c) et d) pour effectuer une mesure sur N autre(s) échantillon(s) biologique(s) où N est un entier naturel supérieur ou égal à l'unité.

10. Procédé selon la revendication précédente, **caractérisé en ce qu'**il comprend, après la mise en oeuvre de l'étape i"), les étapes suivantes :
e₂') exercer une pression sur une autre cartouche (CART2) pour libérer un autre fluide d'étalonnage dans la zone de transport (ZT) afin qu'elle rejoigne la zone de mesure (ZM), cet autre fluide d'étalonnage étant identique au fluide d'étalonnage employé au cours de l'étape e),
f'₂') attendre que la zone de mesure (ZM) soit immergée dans le fluide d'étalonnage, et
g₂') effectuer un autre étalonnage au niveau de la zone de mesure (ZM).

11. Procédé selon la revendication précédente, **caractérisé en ce qu'**il comprend, après avoir évacué l'autre fluide d'étalonnage de la zone de mesure à l'issue de la mise en oeuvre de l'étape g₂'), les étapes suivantes :
e₂") exercer une pression contre une autre cartouche (CART4) pour libérer encore un autre fluide d'étalonnage dans la zone de transport (ZT) afin qu'elle rejoigne la zone de mesure (ZM), cet autre fluide d'étalonnage étant différent du fluide d'étalonnage employé au cours de l'étape e),
f₂") attendre que la zone de mesure (ZM) soit immergée par cet autre fluide d'étalonnage,
g₂") effectuer une mesure sur cet autre fluide d'étalonnage, au niveau de la zone de mesure (ZM).

12. Procédé selon la revendication précédente, **caractérisé en ce que** le fluide d'étalonnage employé à l'étape e₂") est identique au fluide d'étalonnage employé à l'étape e₁").
